# EUROPEAN PATENT SPECIFICATION

(11) **EP 2 288 699 B1**
(45) Date of publication and mention of the grant of the patent: **28.12.2011**
(21) Application number: 09761298.0
(22) Date of filing: 21.05.2009
(51) Int. Cl.: C12N 11/14, A61L 27/08

(54) **METHOD OF MAKING ARRANGED CELL STRUCTURES**
VERFAHREN ZUR HERSTELLUNG VON ANGEORDNETEN ZELLSTRUKTUREN
PROCÉDÉ DE FABRICATION DE STRUCTURES CELLULAIRES ORGANISÉES

(30) Priority: 10.06.2008 CZ 20080355
(43) Date of publication of application: 02.03.2011
(73) Proprietor: Institute Of Physics, As Cr, V. V .i., 182 21 Praha 8 (CZ); Institute Of Physiology Of AS CR, V.V.I., 142 20 Praha 4 (CZ); Charles University In Prague, 121 08 Praha 2 (CZ)
(72) Inventor: REZEK , Bohuslav, 182 00 Praha 8 (CZ); MICHALIKOVA , Lenka, 951 36 Nove Mesto nad Vahom (SK); KROMKA, Alexander, 951 36 Lehota (SK); KALBACOVA, Marie, 468 11 Janov nad Nisou (CZ); KMOCH, Stanislav, 110 00 Praha 1 (CZ); GRAUSOVA, Lubica, 841 01 Bratislava (SK); BACAKOVA , Lucie, 155 00 Praha 5 (CZ); VANECEK, Milan, 143 00 Praha 12 (CZ); KOCKA, Jan, 190 16 Praha 9 (CZ)
(74) Representative: Beetz & Partner
(86) International application number: PCT/CZ2009/000072
(87) International publication number: WO 2009/149673

(56) References cited:
- US-A1- 2005 214 535
- BACAKOVA ET AL: "Improved adhesion and growth of human osteoblast-like MG 63 cells on biomaterials modified with carbon nanoparticles" DIAMOND AND RELATED MATERIALS, ELSEVIER SCIENCE PUBLISHERS, AMSTERDAM, NL, vol. 16, no. 12, 20 November 2007 (2007-11-20), pages 2133-2140, XP022354290 ISSN: 0925-9635
- ISMAIL F S M ET AL: "The influence of surface chemistry and topography on the contact guidance of MG63 osteoblast cells" JOURNAL OF MATERIALS SCIENCE: MATERIALS IN MEDICINE 200705 NL, vol. 18, no. 5, May 2007 (2007-05), pages 705-714, XP019503686 ISSN: 0957-4530

## Description

### Technical field

The invention relates to the controlled organization of cells into patterns on the solid surfaces

### Background art

Controlled organization of cells is a principle attractive not only for medicine or pharmacology in general, but also for nano-biotechnology or bio-electronics, which have been becoming more and more important fields in the recent years. Nanotechnologies often require controlled structuring and engineering of both material and chemical properties in the order of micro- and nanometers, which can be exploited for the fabrication of functional devices, for the selective growth and organization of structures as well as for the preparation of heterogeneous interfaces between organic and inorganic materials. Such hybrid devices can create a bridge between the organic and inorganic world, combining their advantages and being beneficial for meeting the today's high requirements on miniaturization, high performance, and new functions. The fabrication of microscopic templates for the attachment of carbon nanotubes, DNA molecules, cells and cellular membranes on a particular spot on an electronic chip could serve as a perfect example of such devices. Moreover, various ways of the organization of cells are being exploited in tissue engineering.

For the oriented attachment of cells onto a substrate, mainly ligand-receptor bonds are utilized. For example, peptides, containing an amino acid sequence RGD, distinguishable by various types of intengrin cell receptors, can be covalently bound to the surface. Subsequently, cell attached onto these peptides forms focal adhesions (or focal adhesion plaques composed from many structural and signal proteins, which the cell requires for the interaction with the substrate's surface). This type of interaction leads to the transfer of information between the cell and the substrate and the cell reacts by starting various life programs (proliferation, differentiation, death, etc.). Furthermore, "printing" of these RGD peptides in various distances and patterns leads to a regionally selective adhesion of cells. Other molecules able to mediate adhesion, such as P-selectin, whole colagen fibers or other extracellular matrix proteins which are commercially available can also be utilized. Other possibilities include e.g. self-organized monolayers of organic chains which are covalently terminated with -CH₃, -NH₂ and other groups.

Apart from the protein layer between cell and substrate, the substrate itself and its properties play a major role in cell adhesion. The substrate and its properties stimulate the cell reaction (response) to the entire environment, i.e. whether they can adhere, proliferate, differentiate or, on the other hand, whether they cannot adhere and die.

Various materials can be exploited as a substrate for the growth and organization of the cells. They can be, depending on the particular application, either flat with varying degrees of roughness or shaped into complex three-dimensional structures. From the point of view of their electronic properties, largely metals (titanium compounds) or dielectrics (insulators), such as inorganic oxides (SiO₂, TiO₂) or various polymers (polystyrene - PS, or polymethylmatelcrylate - PMMA), are used. Recently, however, the use of semiconductors, whose conductive properties can be easily tuned to allow the fabrication of integrated circuits and biological structures based only on one type of material, has come to focus. In industry, silicon together with its oxides is the most widespread element. It has countless applications in microelectronics, optoelectronics, photovoltaics, xerography, sensors, etc. Emerging semiconductive materials include wide-band-gap semiconductors such as diamond or silicon carbide. Especially diamond offers a unique combination of semiconductive, mechanical, chemical and biological properties. Although diamond is a semiconductor with wide energy band gap (5.5 eV), which makes it an insulator in its intrinsic state, it can be doped with impurities (boron, phosphorus, etc.) in order to obtain p- or n-type conductivity. Moreover, a two-dimensional highly conductive layer can be generated on hydrogen-terminated diamond surfaces. Due to the wide band gap, diamond is optically transparent, which is important for the optical detection of biological properties. Furthermore, it is hard, and mechanically, chemically and physically stable. Its unusually wide electrochemical window (>3 V), in which the surface does not chemically react itself but supports chemical reactions, is highly advantageous for electrochemical measurements, such as impedance spectroscopy, a widely used technique in bio-sensors. Last but not least, diamond is considered highly biologically compatible, since it is actually made up of carbon. Consequently, its possible applications in prosthetics and bio-sensors are intensely studied. Diamond can be synthesized, either as bulk or in the form of thin films deposited on various substrates, using methane decomposition in a plasma discharge.

All in all, a material which firstly enables a controlled organization of cells into structures on surfaces, secondly can be prepared by simple procedures and at low cost, and thirdly does not influence the biological behavior of adhered cells (and consequently ensures the right function and use of the prepared system) is highly desirable for biological applications. The most important parameter of any substrate is naturally its bio-compatibility, particularly the way it influences cell adhesion, growth and survival. Besides biocompatibility, mechanical and chemical resistance plays an important role. The use of special peptides and proteins for the selective adhesion and orientation of cells is relatively difficult and costly and, moreover, their bonding to and stability on the substrate needs to be considered.

### Disclosure of the invention

The present invention provides a method which both overcomes the above-mentioned limitations and meets the requirements by utilizing diamond as a substrate for the organization of cells. The main and unique advantage of diamond is that it combines excellent semiconductive, mechanical, chemical, and biological properties. From the viewpoint of semiconductors, its free-carrier mobility is higher than in silicon by several orders of magnitude. What is more, it is composed of carbon, a natural interface for organic materials. Last but not least, its both chemical and hard-radiation and high-temperatures resistance complements all the highly advantageous properties for biological and bio-electronic applications.

Diamond can be either natural or synthetic, prepared at high pressure and temperatures or by plasma-enhanced chemical vapor deposition or by the detonation of explosives.

Diamond can be either intrinsic or impurity (boron, phosphorus) doped, the concentration of the impurities is not limited.

At the same time, the surface of diamond can be terminated with carbon or oxygen or hydrogen or nitrogen or halogen atoms (fluorine, chlorine) or their compounds or with a combination of these species. The concentration of the terminating species is not limited.

The method of the preparation of organized cell patterns lies in the spontaneous adhesion of cells suspended in a cultivation medium onto a surface of a solid, preferably diamond. Prior to adhesion, hydrophilic areas separated with hydrophobic areas need to be fabricated on the surface of the solid. The hydrophobic areas are fabricated through their exposure to hydrogen plasma at at least 400 °C, while the exposure to oxygen radicals from plasma discharge or ultraviolet radiation leads to the formation of hydrophilic areas. The solid with such a chemically patterned surface is immersed in the cultivation medium with cells with the ability to form bone or neural tissue, such as human bone-forming cells and mesenchymal (stem) cells able to differentiate into bone-forming cells or neurons.

In addition, the surface of the solid (diamond) can be structured morphologically. Diamond surfaces can be of different surface roughness depending on the selected regime of growth and at the same time three-dimensional structures can be fabricated.

### Example

520-µm-thick boron-doped (conductivity 0.1 (Ωcm)⁻¹) silicon substrate is ultrasonically cleaned in isopropanol for 5 minutes, then it is dipped into deionized water and dried with a flow of dry nitrogen. Water-suspended nano-diamond powder with the nominal size of particles of 5 nm is then deposited onto the clean substrate in an ultrasonic bath. The deposition takes 40 minutes, leading to the formation of a 5- to 25-nm-thin and as much as from 80 % continual layer of nano-diamond powder.

A layer of nano-crystalline (NCD) diamond with the thickness in the order of several hundreds of nanometers is later deposited on this starting layer by microwave-plasma-enhanced chemical vapor deposition. 1 % methane dilution in hydrogen is used as the gas, other deposition parameters include the pressure of 3000 Pa, flow rate of 5x10⁻⁶ m³/s, plasma generator output of 1200 W and substrate temperature of 800 °C. The resulting thickness of the NCD layer is around 150 nm.

After the deposition, the layer is boiled in a mixture of acids (H₂SO₄ + KNO₃, ratio 3: 1), rinsed with deionized water and dried with a flow of dry nitrogen. Afterwards, the layer is treated with oxygen r.f. plasma with the output of 300 W for 3 minutes and finally it is terminated with hydrogen in hydrogen plasma at 800 °C for 10 minutes. This procedure yields clean and well-defined surface of diamond, which is terminated with hydrogen and highly hydrophobic (contact angle - 90°).

The microscopic templates for the organization of cells on the NCD layer are fabricated using a lithographic mask (optical or electron lithography). The layers are exposed to high-frequency oxygen plasma (300 W) for 3 minutes with this mask, then the mask is rinsed from the surface of diamond using acetone and water, eventually other solvents. After subsequent drying the layer is prepared for cell-seeding.

Prior to seeding, the NCD layers are sterilized with UV irradiation or alcohol for 10 minutes. Silicon substrates with diamond layers are placed at the bottom a cultivation well-plates. Cells are seeded on diamond from a concentrated solution with typical concentrations of 1000-10000 cells/cm² and cultivated in McCoy's 5A medium, containing 0-15% fetal bovine serum (FBS) and antibiotics (penicillin, streptomycin). Cells are then cultivated under standard conditions for tissue cultures in an incubator at 37 °C in 5 % CO₂ for two days.

Examples of structures fabricated using the above-described procedure are 30- to 200-µm-wide oxygen-terminated stripes separated by hydrophobic hydrogen-terminated areas. Cell line of transformed human bone-forming cells (osteoblasts) SAOS-2 was used for the demonstration of the cell organization. The cells preferentially adhered and proliferated on the oxygen-terminated diamond with sharp borders on the H/O interface. Moreover, cells adhered on narrow stripes were organized into chain-like patterns along the stripes.

### Industrial applicability

The described method for the making of arranged cell structures can be applied in medicine, pharmacy, biology and electronics. It can also be exploited in tissue engineering, optical, electronic and chemical bio-sensors, bio-stimulators of growth and cell differentiation, bio-electronic signal transmitters, for the "micro-arrays" technology for modem proteomics and genomics, etc.

## Claims

1. The method of making arranged cell structures by attaching cells on the surface of a solid, **characterized in that** the above-mentioned solid is diamond having hydrophilic areas separated by hydrophobic areas fabricated on its surface and, when its surface is chemically structured in the above-mentioned way, said solid is exposed to a cultivation medium comprising cells.

2. The method according to claim 1, **characterized in that**, in addition, protrusions or troughs are fabricated on the diamond surface.

3. The method according to claims 1 or 2, **characterized in that** the diamond is prepared by the deposition of diamond powder on a substrate cleaned in an ultrasonic bath.

4. The method according to claim 3 **characterized in that** the diamond powder is mixed with a polymer and this polymer composite is deposited onto the predefined
areas using a lithographic photoresist technique or a lift-off process.

5. The method according to claims 3 or 4, **characterized in that** an additional diamond layer is deposited on the first diamond layer using microwave-plasma-enhanced chemical vapor deposition.

6. The method according to claims 1 or 2 or 3 or 4, **characterized in that** the diamond is of natural origin.

7. The method according to claim 5, **characterized in that** the diamond is doped with impurities.

8. The method according to claims 1 or 2 or 3 or 4 or 5 or 6 or 7, **characterized in that** in order to fabricate the hydrophobic areas the surface of diamond is exposed to hydrogen plasma at at least 400 °C and in order to fabricate the hydrophilic areas the surface is exposed to oxygen radicals created by a plasma discharge or ultraviolet radiation.

9. The method according to claims 1 or 2 or 3 or 4 or 5 or 6 or 7 or 8, **characterized in that** the cultivation medium contains human osteoblasts, mesenchymal stem cells which are able to form bone tissue or neurons after differentiation.

## Patentansprüche

1. Verfahren zur Herstellung von angeordneten Zellstrukturen durch Anlagern von Zellen auf der Oberfläche eines Feststoffs, **dadurch gekennzeichnet, dass** der vorgenannte Feststoff ein Diamant ist, welcher hydrophile Bereiche aufweist, die durch hydrophobe Bereiche getrennt sind, die auf seiner Oberfläche ausgebildet sind, und der Feststoff, wenn seine Oberfläche auf die vorgenannte Weise chemisch strukturiert ist, einem Züchtungsmedium mit Zellen ausgesetzt wird.

2. Verfahren nach Anspruch 1, **dadurch gekennzeichnet, dass** zusätzlich Vorsprünge oder Vertiefungen auf der Diamantoberfläche ausgebildet sind.

3. Verfahren nach Anspruch 1 oder 2, **dadurch gekennzeichnet, dass** der Diamant durch die Ablagerung von Diamantpulver auf einem in einem Ultraschallbad gereinigten Substrat hergestellt wird.

4. Verfahren nach Anspruch 3, **dadurch gekennzeichnet, dass** das Diamantpulver mit einem Polymer vermischt und dieser Polymerverbundstoff auf den vordefinierten Bereichen unter Anwendung einer lithografischen Fotoresisttechnik oder eines Abziehvorgangs abgeschieden wird.

5. Verfahren nach Anspruch 3 oder 4, **dadurch gekennzeichnet, dass** eine zusätzliche Diamantschicht auf der ersten Diamantschicht unter Anwendung einer mikrowellenplasmaverstärkten chemischen Dampfphasenbeschichtung abgeschieden wird.

6. Verfahren nach Anspruch 1 oder 2 oder 3 oder 4, **dadurch gekennzeichnet, dass** der Diamant natürlichen Ursprungs ist.

7. Verfahren nach Anspruch 5, **dadurch gekennzeichnet, dass** der Diamant mit Fremdstoffen dotiert ist.

8. Verfahren nach den Ansprüchen 1 oder 2 oder 3 oder 4 oder 5 oder 6 oder 7, **dadurch gekennzeichnet, dass** zur Herstellung der hydrophoben Bereiche die Oberfläche des Diamanten Wasserstoffplasma bei mindestens 400°C ausgesetzt wird und zur Herstellung der hydrophilen Bereiche die Oberfläche Sauerstoffradikalen ausgesetzt wird, die durch eine Plasmaentladung oder Ultraviolettbestrahlung erzeugt werden.

9. Verfahren nach den Ansprüchen 1 oder 2 oder 3 oder 4 oder 5 oder 6 oder 7 oder 8, **dadurch gekennzeichnet, dass** das Züchtungsmedium menschliche Knochenbildner enthält, nämlich mesenchymale Stammzellen, die nach einer Differenzierung Knochengewebe oder Neuronen bilden können.

## Revendications

1. Procédé de fabrication de structures cellulaires organisées en fixant des cellules à la surface d'un solide, **caractérisé en ce que** le solide mentionné ci-dessus est un diamant possédant des zones hydrophiles séparées par des zones hydrophobes fabriquées sur sa surface et, quand sa surface est structurée chimiquement de la manière mentionnée ci-dessus, ledit solide est exposé à un milieu de culture comprenant des cellules.

2. Le procédé selon la revendication 1, **caractérisé en ce que**, en outre, des saillies ou des creux sont fabriqués sur la surface du diamant.

3. Le procédé selon les revendications 1 ou 2, **caractérisé en ce que** le diamant est préparé en déposant une poudre de diamant sur un substrat nettoyé dans un bain à ultrasons.

4. Le procédé selon la revendication 3, **caractérisé en ce que** la poudre de diamant est mélangée avec un polymère et ce composite à base de polymère est déposé sur les zones définies au préalable en utilisant une technique photolithographique ou un procédé de décollement.

5. Le procédé selon les revendications 3 ou 4, **caractérisé en ce qu'**une couche de diamant supplémentaire est déposée sur la première couche de diamant en utilisant un dépôt chimique en phase vapeur assisté par plasma micro-ondes.

6. Le procédé selon les revendications 1, 2, 3 ou 4, **caractérisé en ce que** le diamant est d'origine naturelle.

7. Le procédé selon la revendication 5, **caractérisé en ce que** le diamant est dopé avec des impuretés.

8. Le procédé selon les revendications 1, 2, 3, 4, 5, 6 ou 7, **caractérisé en ce que**, pour fabriquer les zones hydrophobes, la surface de diamant est exposée à du plasma d'hydrogène à au moins 400°C et, pour fabriquer les zones hydrophiles, la surface est exposée à des radicaux d'oxygène créés par une décharge de plasma ou un rayonnement ultraviolet.

9. Le procédé selon les revendications 1, 2, 3, 4, 5, 6, 7 ou 8, **caractérisé en ce que** le milieu de culture contient des ostéoblastes humains, des cellules souches mésenchymateuses qui peuvent former du tissu osseux ou des neurones après différenciation.
